# FASCICULE DE BREVET EUROPEEN

(11) **EP 0 886 654 B1**
(45) Date de publication et mention de la délivrance du brevet: **30.05.2001**
(21) Numéro de dépôt: 97914388.0
(22) Date de dépôt: 13.03.1997
(51) Int. Cl.: C08B 37/16, A61K 47/40

(54) **THIOUREIDO-CYCLODEXTRINES, UTILISABLES EN PARTICULIER POUR SOLUBILISER DES AGENTS ANTITUMORAUX ET ANTIPARASITAIRES ET LEURS PROCEDES DE PREPARATION**
THIOUREIDO CYCLODEXTRINEN,INSBESONDERE ZUR SOLUBILISIERUNG VON ANTITUMOREN UND ANTIPARASITAEREN MITTELN UND VERFAHREN ZUR HERSTELLUNG
THIOUREIDO-CYCLODEXTRINS PARTICULARLY USEFUL FOR SOLUBILISING ANTITUMORAL AND ANTIPARASITIC AGENTS, AND METHODS FOR PREPARING SAME

(30) Priorité: 14.03.1996 FR 9603221
(43) Date de publication de la demande: 30.12.1998
(73) Titulaire: CENTRE NATIONAL DE LA RECHERCHE SCIENTIFIQUE, 75016 Paris Cédex (FR)
(72) Inventeur: DEFAYE, Jacques, F-38330 Saint-Ismier (FR); ORTIZ-MELLET, Carmen, E-41011 Séville (ES); GARCIA-FERNANDEZ, José-Manuel, E-41011 Séville (ES)
(74) Mandataire: Des Termes, Monique
(86) Numéro de dépôt international: FR9700449
(87) Numéro de publication internationale: WO9733919

(56) Documents cités:
- Aucun document pertinent relevé

## Description

La présente invention a pour objet de nouveaux dérivés de cyclodextrines, des thiouréido-cyclodextrines, qui sont utilisables en particulier pour solubiliser dans un milieu aqueux des agents anti-tumoraux et antiparasitaires, notamment ceux de la famille du Taxol.

Les cyclodextrines, ou cyclomaltooligosaccharides, sont des oligosaccharides cycliques qui ont la propriété d'inclure dans leur cavité des molécules diverses, de taille adpatée à celle de la structure hôte. Le caractère généralement apolaire de ces associations conduit à inclure préférentiellement des structures de type hydrophobe, permettant notamment la solubilisation dans l'eau de composés peu ou pas solubles dans ces milieux.

La solubilité relativement faible dans l'eau des cyclodextrines, et notamment de la plus accessible d'entre elles sur le plan économique, la β-cyclodextrine (18 g/l à 25°C) limite cependant leur utilisation dans ce but.

Pour remédier à cette situation, on a modifié chimiquement les cyclodextrines pour améliorer leur solubilité dans l'eau. Ainsi, on a décrit dans les documents WO-A-95/19994, WO-A-95/21870 et EP-A-0 403 366 des dérivés ramifiés de cyclodextrines comportant un ou plusieurs substituants monosaccharidiques ou oligosaccharidiques liés à la cyclodextrine par un atome d'oxygène ou de soufre ainsi que leur utilisation. Ces cyclodextrines ramifiées sont en particulier susceptibles de se combiner avec le Taxol et ses dérivés, en particulier le Taxotère®, qui sont des agents antitumoraux et antiparasitaires, comme il est décrit par P. Potier dans Chem. Soc. Rev., 21, 1992, pp. 113-119, M.C. Bissery et al dans Cancer Research, 51, 1991, pp. 4845-4852 et J. Schrével et al dans Proc. Natl. Acad. Sci. USA, vol 91, pp. 8472-8476, 1994. On obtient ainsi des complexes d'inclusion, ce qui permet de solubiliser dans l'eau ces agents antitumoraux. A titre d'exemple, la solubilité dans l'eau du Taxotère® qui est 0,004 g/l, peut être amenée jusqu'à 6,5 g/l par encapsulation dans le 6^{I}-S-α-maltosyl-6^{I}-thiocyclomaltoheptaose, comme il est décrit dans WO-A-95/19994.

Le document EP-A-0 605 753 décrit des complexes d'inclusion du Taxol utilisant des cyclodextrines ramifiées telles que les maltosyl-cyclodextrines, pour augmenter la solubilité du Taxol dans l'eau.

Des dérivés de cyclodextrines comportant un ou plusieurs substituants glycosyle ou maltosyle liés à la cyclodextrine par un atome de soufre sont également décrits par V. Lainé et al dans J. Chem. Soc., Perkin Trans, 2, 1995, pp. 1479-1487. Ces dérivés ont été testés pour solubiliser une substance active telle que la prednisolone.

La présente invention a pour objet d'autres dérivés de cyclodextrines présentant non seulement un intérêt pour la solubilisation de substances actives, en particulier d'agents antitumoraux et antiparasitaires de la famille du Taxol, mais permettant de plus un ciblage et une vectorisation de la substance active sur les organes à traiter.

Les nouveaux dérivés de cyclodextrines sont des thiouréido-cyclodextrines répondant à la formule : 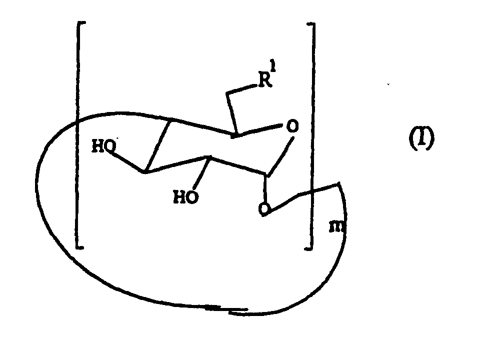 dans laquelle m est égal à 6, 7 ou 8 et les R¹ qui peuvent être identiques ou différents, représentent OH ou NH-CS-NHR² avec R² représentant un groupe alkyle, un groupe dérivé d'un monosaccharide ou d'un oligo saccharide éventuellement substitué, un groupe dérivé d'un glycosyl-aminoacide ou un groupe dérivé d'un glycopeptide, à condition que l'un au moins des R¹ représente NH-CS-NHR².

Dans ces nouveaux dérivés, la présence d'une liaison ou d'un espaceur de type thiourée -NH-CS-NH- au lieu d'un atome d'oxygène ou de soufre pour lier le subtituant R² est intéressante, notamment pour associer la cyclodextrine à un motif hydrophile tel qu'un dérivé glucidique, en raison de la simplicité de la réaction de couplage. Par ailleurs, les thiourées sont des composés de grande stabilité et de structure bien définie qui peuvent être couplées à de nombreux substituants, comme on le verra plus loin. Des composés de type thiourée ont d'ailleurs été utilisés pour la préparation de néo-glycoconjugués comme il est décrit par Z.J. Witczak, Adv. Carbohydr. Chem., 44, 1986, pp 91-145 et C.M. Reichert, C.E. Hayes et I.J. Goldstein, Methods Enzymol., 242, 1994, pp. 108-117).

Les thiouréido-cyclodextrines répondant à la formule (I) donnée ci-dessus peuvent être des cyclodextrines monosubstituées, persubstituées ou partiellement substituées en position alcool primaire, selon le nombre de R¹ représentant NH-CS-NHR². Par ailleurs, le (s) substituant(s) R² peuvent être de divers types.

Ainsi R² peut représenter un groupe alkyle de 1 à 10 atomes de carbone, par exemple le groupe méthyle. R² peut aussi représenter des groupes dérivés de monosaccharides ou d'oligosaccharides éventuellement substitués. A titre d'exemple de groupes dérivés de monosaccharides, on peut citer les groupes dérivés du glucose et du galactose, sous forme α ou β. Dans le cas où le groupe dérivé de monosaccharide est substitué, un ou plusieurs des groupes hydroxyle du monosaccharide peuvent être remplacés par des groupes alcoxy de 1 à 16 atomes de carbone, des groupes acyloxy comme le groupe acétoxy, des groupes amines et amides. Les groupes dérivés d'oligosaccharides peuvent être les groupes maltosyle, maltotriosyle, lactosyle, ou encore de tri- ou tétrasaccharides marqueurs d'affinité cellulaire du type Lewis X ou Sialyl Lewis X, ou encore des oligosaccharides dérivés de l'héparine. Ils peuvent également être substitués par des groupes alcoxy, acyloxy, ou aminés.

Selon l'invention R² peut également représenter un groupe dérivé d'un glycosyl-aminoacide ou d'un glycopeptide.

Les aminoacides susceptibles d'être utilisés sont en particulier les aminoacides naturels tels que l'alanine, la cystéine, l'acide aspartique, l'acide glutamique, la phénylalanine, la glycine, l'histidine, l'isoleucine, la lysine, la leucine, la méthionine, l'asparagine, la proline, la glutamine, l'arginine, la sérine, la thréonine, la valine, le tryptophane, et la tyrosine. On peut aussi utiliser des acides aminés modifiés.

Dans le cas où le groupe R² comprend un peptide, celui-ci peut être formé à partir des acides aminés décrits ci-dessus et comprendre également des acides aminés modifiés.

La présence d'un groupe R² du type glycosyl-aminoacide ou glycopeptide permet de conférer à la cyclodextrine une affinité particulière pour certains sites biologiques car ce groupe peut jouer le rôle de marqueur de reconnaissance externe moléculaire et cellulaire. Ainsi, cette modification de la cyclodextrine peut permettre le ciblage et la vectorisation d'une substance active incluse dans la cyclodextrine.

Les dérivés de cyclodextrine de l'invention peuvent être préparés par deux voies différentes. La première voie (procédé A) consiste à faire réagir une isothiocyanato-cyclodextrine avec une amine comportant le groupe R². La seconde voie (procédé B) consiste à faire réagir une amino-cyclodextrine avec un dérivé comportant une fonction isothiocyanate comportant le groupe R².

Aussi, la présente invention a pour objet un premier procédé (procédé A) de préparation d'une thiouréido-cyclodextrine répondant à la formule (I) donnée ci-dessus, qui consiste à faire réagir une isothiocyanato-cyclodextrine de formule : 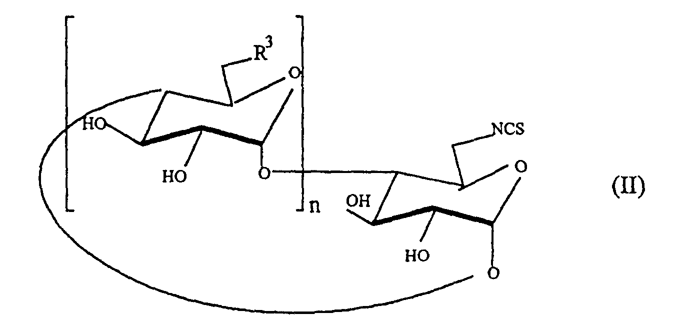 dans laquelle les R³ qui peuvent être identiques ou différents, représentent NCS ou OH et n est égal à 5, 6 ou 7, avec une amine de formule R²-NH₂.

Cette réaction peut être effectuée dans un solvant organique tel que la pyridine.

L'isothiocyanato-cyclodextrine de formule (II) utilisée comme produit de départ dans ce procédé peut être préparée par réaction de l'amino-cyclodextrine correspondante avec du thiophosgène. On peut utiliser pour cette préparation le procédé décrit par J.M. Garcia Fernandez et al dans Carbohydr. Res., 268, 1995, pp 57-71.

Le second procédé (procédé B) de préparation des thiouréido-cyclodextrines de formule (I) de l'invention consiste à faire réagir une amino-cyclodextrine de formule : 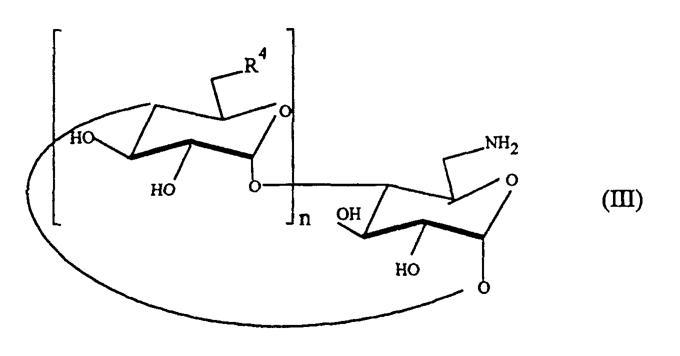 dans laquelle les R⁴ qui peuvent être identiques ou différents, représentent OH ou NH₂, et n est égal à 5, 6 ou 7,
avec un isothiocyanate de formule : R²-NCS dans laquelle R² a la signification donnée ci-dessus.
dans laquelle les R⁴ qui peuvent être identiques ou différents, représentent OH ou NH₂, et n est égal à 5, 6 ou 7,
avec un isothiocyanate de formule : R²-NCS dans laquelle R² a la signification donnée ci-dessus.

Cette réaction peut être effectuée dans un solvant organique tel que la pyridine.

Les amino-cyclodextrines de départ de formule (III) peuvent être préparés par le procédé décrit par J. M. Garcia Fernandez et al dans le document Carbohydr. Res., 268, 1995, pp. 57-71, mentionné ci-dessus.

Lorsque R² est un groupe dérivé d'un monosaccharide, d'un oligosaccharide, d'un glycosyl-aminoacide ou d'un glycopeptide, l'isothiocyanate de formule R²-NCS peut être préparé par réaction du thiophosgène sur un aminodésoxyglycose ou une glycosylamine.

Dans le cas du procédé A, lorsqu'on utilise comme réactif une amine de formule : R²-NH₂ dans laquelle R² représente un groupe dérivé d'un monosaccharide, d'un oligosaccharide, d'un glycosyl-amino-acide ou d'un glycopeptide, cette amine peut être préparée par le procédé décrit par J. M. Garcia Fernandez et al dans J. Org. Chem., 58, 1993, pp. 5192-5199.

Les procédés décrits ci-dessus pour l'obtention des thiouréido-cyclodextrines de l'invention sont très intéressants car ils permettent d'obtenir les dérivés voulus avec des rendements élevés.

Les thiouréido-cyclodextrines de l'invention sont utilisables en particulier pour solubiliser en milieu aqueux des composés chimiques hydrophobes, notamment des agents anti-tumoraux appartenant à la famille du Taxol.

Aussi, l'invention concerne également les complexes d'inclusion des thiouréido-cyclodextrines de formule (I) avec un composé chimique, en particulier hydrophobe, tel qu'une molécule pharmaceutiquement active.

De préférence, dans ces complexes d'inclusion, le composé chimique est un agent antitumoral ou antiparasitaire, notamment de la famille du Taxol comme le Taxol et le Taxotère®.

Ces complexes d'inclusion peuvent être préparés par des procédés classiques, par exemple en ajoutant à une solution ou à une suspension de la thiouréido-cyclodextrine de formule (I) utilisée, le composé chimique en solution ou à l'état pur. On peut ensuite isoler le complexe d'inclusion ainsi formé par lyophilisation.

Dans le cas où on ajoute le composé en solution, par exemple l'agent antitumoral de la famille du Taxol, on utilise une solution concentrée du composé dans un solvant organique miscible à l'eau, par exemple l'acétone, et on soumet ensuite le mélange obtenu à une agitation et à un barbotage de gaz inerte tel que l'azote, pour éliminer le solvant organique.

Dans le cas des composés de la famille du Taxol tels que le Taxotère, on peut aussi disperser ce produit à l'état pur dans une solution stérile d'une thiouréido-cyclodextrine conforme à l'invention.

L'invention a encore pour objet une composition pharmaceutique comprenant un complexe d'inclusion d'un dérivé de cyclodextrine de formule (I) et d'une molécule pharmacologiquement active telle qu'un agent antitumoral ou antiparasitaire, avec un véhicule pharmacologiquement acceptable.

Ces compositions pharmaceutiques qui peuvent être administrée par voie orale ou parentérale, sont par exemple des solutions, des poudres, des suspensions etc., en particulier des solutions injectables.

D'autres caractéristiques et avantages de l'invention apparaitront mieux à la lecture des exemples suivants donnés bien-entendu à titre illustratif et non limitatif.

Ces exemples se rapportent à des thiouréido-cyclodextrines répondant à la formule suivante :

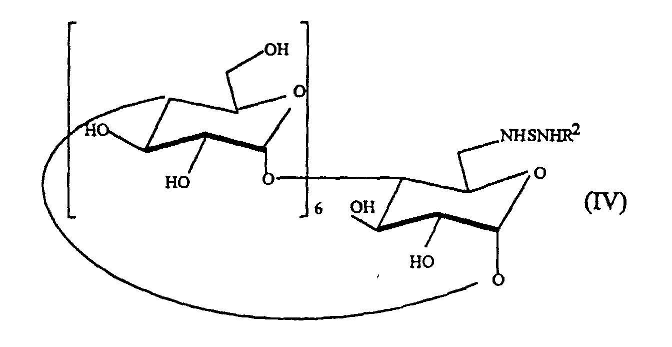

### Exemple 1 : Préparation du 6^{I}-désoxy-6^{I}-(N'-méthylthioureido)cyclomaltoheptaose (composé n°1)

Ce composé répond à la formule (IV) donnée ci-dessus avec R² = CH₃.

Ce composé est obtenu par condensation du 6^{I}-amino-6^{I}-désoxycyclomaltoheptaose avec le méthyl isothiocyanate (procédé B).

### 1. Préparation du 6^{I}-amino-6^{I}-désoxycylomaltoheptaose.

A une solution de 6^{I}-azido-6^{I}-désoxycyclomaltoheptaose (R.C. Petter, J.S Salek, C.T. Sikorski, G. Kumaravel et F.-T. Lin, J. Am. Chem. Soc., 112, 1990, pp 3860-3868) dans le *N,N*-diméthylformamide (DMF, 10 ml), on ajoute le 1,3-propanedithiol (0,62 ml, 6,15 mmol) et l'éthyldiisopropylamine (0,65, ml, 3,69 mmol). Le mélange réactionnel est agité à température ambiante pendant 16 h, puis additionné d'acétone (100 ml). Le précipité obtenu est filtré, lavé avec de l'acétone, redissous dans l'eau (100 ml) et filtré sur Celite. Après lyophilisation de la solution aqueuse, on obtient 1,34 g de l'amine (rendement 92 %) ayant les données de ¹³C RMN décrites dans la littérature (S.E. Brown, J. H. Coates, D.R. Coghlan, C.J. Easton, S.J. van Eyk, W. Janowski, A. Lapore, S.F. Lincoln, Y. Luo, B.L. May, D.S. Schiesser, P. Wang et M.L. Williams, Aust. J. Chem., 46, 1993, pp. 953-958).

### 2. Préparation du composé n°1.

A une solution du 6^{I}-amino-6^{I}-désoxycyclomaltoheptaose obtenu en 1 (100 mg, 0,09 mmol) dans de la pyridine (2 ml), on ajoute l'isothiocyanate de méthyle (8 mg, 0,1 mmol) et le mélange réactionnel est maintenu pendant 48 h à température ambiante, puis concentré. Le résidu obtenu est dissous dans l'eau (10 ml), agité avec du chloroforme (2 x 10 ml), la phase organique est décantée pour séparer l'excès d'isothiocyanate de méthyle et la phase aqueuse est lyophilisée. On obtient ainsi 103 mg du composé n°1 (rendement 95 %) pur à > 90 % (CLHP). Après purification par CLHP (colonne Nucléosil C-18, 5 µ; éluant MeOH-eau 12:88 v/v), on obtient le composé n°1 (95 mg, 87%) ayant les caractéristiques suivantes :
- [α]_{D} + 108,7° (c, 1,03, eau) ;
- spectre de masse (FAB⁺) : *m/z* 1229,2 (96 %, [M + Na]⁺), 1207,4 (100, [M+H]⁺).
- solubilité dans l'eau : 775 g/l, 640 mM.

### Exemple 2 : Préparation du 6^{I}-désoxy-6^{I}-[3-(méthyl-α-D-glucopyranosid-6-yl)thiouréido)]cyclomaltoheptaose (composé n°2)

Ce composé répond à la formule (IV) avec R² répondant à la formule (V) :

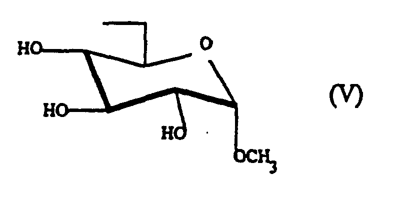

Ce composé est préparé par condensation du 6^{I}-désoxy-6^{I}-isothiocyanatocyclomaltoheptaose avec le méthyl 6-amino-6-désoxy-α-D-glucopyranoside (procédé A)

### 1. Préparation du 6^{I}-désoxy-6^{I}-isothiocyanatocyclomaltoheptaose.

A une solution de 6^{I}-amino-6^{I}-désoxycyclomaltoheptaose (1,13 g, 1 mmol) dans un mélange eau-acétone (3:2, 75 ml) on ajoute le carbonate de calcium (0,9 g, 3 mmol) et le thiophosgène (0,15 ml, 1,5 mmol). La suspension est agitée à température ambiante pendant 16 h, puis concentrée a la moitié de son volume, diluée par l'eau (30 ml) et déminéralisée par agitation avec la résine échangeuse ionique Amberlite MB-6113 (H+, OH- ; 5 ml) pendant 15 minutes. La solution aqueuse est filtrée et lyophilisée. Les caractéristiques de ce produit sont les suivantes :
- [α]_{D} +112,1°(c 0,6, pyridine).

### 2. Préparation du méthyl 6-amino-6-désoxy-α-D-glucopyranoside.

Le méthyl-6-amino-6-désoxy-α-D-glucopyranoside est préparé à partir du méthyl-α-D-glucopyranoside commercial en trois étapes, avec un rendement global de 80 % par le procédé décrit par J. M. Garcia Fernandez, C. Ortiz Mellet et J. Fuentes dans J. Org. Chem., 58, 1993, pp. 5192-5199.

### 3. Préparation du composé 2.

A une solution du 6^{I}-désoxy-6^{I}-isothiocyanato-cyclomaltoheptaose obtenu dans l'étape 1 (100 mg, 85 µmol) dans de la pyridine (3 ml), on ajoute le méthyl 6-amino-6-désoxy-α-D-glucopyranoside obtenu en 2 (16 mg, 85 µmol). Le mélange réactionnel est agité à température ambiante pendant 48 h, puis concentré. Les traces de pyridine sont éliminées par coévaporation avec de l'eau (1 ml). Le résidu est alors repris avec de l'eau et précipité par addition d'éthanol. On obtient ainsi 110 mg du composé n°2 (rendement 95 %) pur à > 90 % (CLHP). Après purification par CLHP (colonne Nucléosil, C-18, 5 µ ; éluant MeOH-eau 12:88 v/v) on obtient le composé n°2 ayant les caractéristiques suivantes :
- [α]_{D} +96,9° (c 0,66, eau) ;
- spectre de masse (FAB⁺): m/z 1391,2 (20 %, [M+Na]⁺), 1369,3 (100, [M+H]+),
- solubilité dans l'eau : 710 g/l, 518 mM.

### Exemple 3 : Préparation du 6^{I}-désoxy-6^{I}-[méthyl-2,3,4-tri-O-acétyl-α-D-glucopyranosid-6-yl)thiouréido)]cyclomaltoheptaose (composé n°3)

Ce composé répond à la formule (IV) avec R² répondant à la formule (VI) : 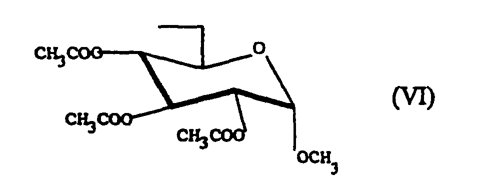 Il est préparé par le procédé B.

A une solution du 6^{I}-amino-6^{I}-désoxycyclomaltoheptaose obtenu dans l'exemple 1 (118 mg, 0,1 mmol) dans de la pyridine (2 ml), on ajoute le méthyl 2,3,4-tri-O-acétyl-6-désoxy-6-isothicyanato-α-D-glucopyranoside préparé selon Garcia Fernandez et al dans J. Org. Chem., 58, 1993, pp. 5192-5199, (36 mg, 0,1 mmol), puis le mélange réactionnel est maintenu pendant 48 h à température ambiante, et concentré. Le résidu est dissous dans l'eau (10 ml), lavé par le chloroforme (2 x 10 ml) et lyophilisé. On obtient ainsi 137 mg du composé n°3 (rendement de 92 %) ayant les caractéristiques suivantes :
- [α]_{D} + 116,1° (c 0,62, eau) ;
- spectre de masse (FAB⁺) : *m/z* 1517,2 (100 %, [M+Na]⁺),
- solubilité dans l'eau : 558 g/l, 373 mM.

### Exemple 4 : Préparation du 6^{I}-désoxy-6^{I}-[3-(N-glycyl-β-D-glucopyranosylamin-6-yl)thiouréido)]cyclomalto-heptaose (composé n°4)

Ce composé répond à la formule (IV) avec R² répondant à la formule :

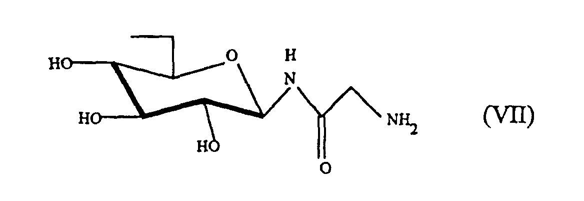

Ce composé est obtenu par condensation du 6^{I}-désoxy-6^{I}-isothiocyanatocyclomaltoheptaose (voir exemple 2) avec la 6-amino-N-(N'-tert-butyloxycarbonyl) glycyl-6-désoxy-β-D-glucopyranosylamine suivie d'une hydrolyse acide du groupement protecteur tert-butyloxycarbonyle (procédé A).

### 1) Préparation de la 6-amino-N-(N'-tert-butyloxycarbonyl)glycyl-6-désoxy-β-D-glucopyranosylamine.

Ce composé est préparé en effectuant les étapes suivantes :

### - a) Préparation de la 6-azido-6-désoxy-β-D-glucopyranosylamine.

A une solution du 6-azido-6-désoxy-D-glucose (K. Dax et al., J. Carbohydr. Chem., 9, 1990, pp. 479-499 ; 4,3 g, 24,3 mmol) dans l'ammoniaque aqueux (16 M, 125 ml), on ajoute le bicarbonate d'ammonium (1,92 g, 24,3 mmol) et le mélange est agité à 40°C pendant 30 h, puis concentré à la moitié de volume, dilué avec de l'eau (150 ml) et lyophilisé. Le produit obtenu (4,3 g, 93 %) contient la 6-azido-6-désoxy-β-D-glucopyranosylamine à > 80 % pure (¹³C RMN) qui est utilisé dans l'étape suivante sans autre purification.

### - b) Préparation de la 6-azido-N-(N'-tert-butyloxycarbonyl)glycyl-6-désoxy-β-D-glucopyranosylamine.

A une solution de N-(tert-butyloxycarbonyl)glycine (66 mg, 0,40 mmol) dans du N,N-diméthylformamide (DMF, 1 ml), on ajoute, successivement, une solution de la 6-azido-6-désoxy-β-D-glucopyranosylamine obtenue en a) (100 mg, 0,49 mmol) dans du DMF (1 ml), une solution de *0*-benzotriazol-1-yl-*N*,*N*,*N'N'*-tétraméthyluronium hexafluorophosphate (HBTU, 0,55 g, 1,47 mmol) dans du DMF (5 ml), de la diisopropyléthylamine (60 mg), et une solution de 1-hydroxybenzotriazole (HOBt, 66 mg, 0,49 mol) dans du DMF (1 ml). Le mélange est agité à température ambiante pendant 16 h, puis concentré et le résidu purifié par chromatographie sur colonne de gel de silice (chloroforme-méthanol 19:1 v/v). On obtient ainsi 90 mg de 6-azido-*N*-(*N*'-tert-butyloxycarbonyl)glycyl-6-désoxy-β-D-glucopyranosylamine (rendement de 55 %) ayant les caractéristiques suivantes :
- [α]_{D} + 3,8° (c 1,1 méthanol).

### - c) Préparation de la 6-amino-N-(N'-tert-butyloxycarbonyl)glycyl-6-désoxy-β-D-glucopyranosylamine

A une solution de la 6-azido-N-(N'-tert-butyloxycarbonyl)glycyl-6-désoxy-β-D-glucopyranosylamine obtenue en b) (0,14 g, 0,37 mmol) dans du DMF (6 ml), on ajoute le 1,3-propanedithiol (0,19 ml, 1,87 mmol) et l'éthyldiisopropylamine (0,19 ml, 1,17 mmol). Le mélange réactionnel est maintenu à température ambiante pendant 3 jours, puis concentré et le résidu est cristallisé dans de l'éthanol-eau. On obtient ainsi 125 mg de l'amine (rendement 92 %) ayant les caractéristiques suivantes :
- [α]_{D} -5°(c,08, eau).

### 2. Préparation du 6^{I}-désoxy-6^{I}-{3'- [N-(N'-tert-butyloxycarbonyl) glycyl-β-D-glucopyranosylamino-6-yl]thiouréido}cyclomaltoheptaose.

A une solution du 6^{I}-désoxy-6^{I}-isothiocyanatocyclomaltoheptaose (105 mg, 0,09 mmol) dans de la pyridine (2 ml), on ajoute la 6-amino-N-(N'-tert-butyloxycarbonyl) glycyl-6-désoxy-β-D-glucopyranosylamine (30 mg, 0,09 mol). Le mélange réactionnel est maintenu à température ambiante pendant 3 jours, puis concentré. Les traces de pyridine sont éliminées par coévaporation avec de l'eau. Le résidu est repris avec de l'eau (1 ml) et précipité par addition d'éthanol. On obtient ainsi 95 mg (rendement 70 %) d'une poudre blanche, présentant les caractéristiques suivantes :
- [α]_{D} +103,4° (c 0,9, eau)
- spectre de masse (FAB+) : *m/z* 1511,2 (100%, [M+H]⁺)

### 3. Préparation du composé n°4.

Une solution du 6^{I}-désoxy-6^{I}-{3'-[*N*-(N'-tert-butyloxycarbonyl) glycyl-β-D-glucopyranosylamine-6-yl]thiouréido}cyclomaltoheptaose (77 mg, 51 µmol) dans l'acide trifluroacétique-eau (9:1 v/v, 1 ml) est maintenue à température ambiante pendant 1 h, puis concentrée. Les traces d'acide trifluoroacétique sont éliminées par coévaporation avec de l'eau. Le résidu est dissous dans l'eau (5 ml), la solution résultante traitée par de la résine échangeuse ionique Amberlite IR-904 (OH⁻, 2 ml), filtrée et lyophilisée. On obtient ainsi 66 mg du composé n°4 (rendement de 91 %) pur à > 95 % (CLHP). Après purification par CLHP, on obtient le composé n°4 (58 mg, 80 %) ayant les caractéristiques suivantes :
- [α]_{D} +82,2° (c 1,8, eau) ;
- spectre de masse (FAB+) : *m/z* 1411,3 (100%, M+H]⁺),
- solubilité dans l'eau : > 800 g/1, 567 mM.

### Exemple 5 : Inclusion du Taxotère dans le 6^{I}-désoxy-6^{I}-(N'-méthylthiouréido)cyclomaltoheptaose (composé n°1)

On part du Taxotère à l'état pur et on disperse 2,1 mg (2,6 µmol) de ce produit dans 3 ml d'une solution contenant 64,2 mmol/l du composé n°1 dans l'eau stérile, puis on agite la suspension obtenue à la température ambiante jusqu'à obtention d'une solution claire qui indique l'encapsulation du Taxotère dans la molécule de cyclodextrine. On obtient ainsi une augmentation importante de la solubilité du Taxotère (4,7 g/l, 5,8 mmol/l), celle-ci étant de l'ordre de 0,004 g/l en l'absence de cyclodextrine.

## Revendications

1. Thiouréido-cyclodextrine répondant à la formule : 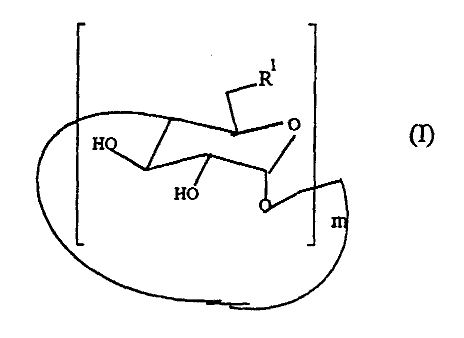 dans laquelle m est égal à 6, 7 ou 8 et les R¹ qui peuvent être identiques ou différents, représentent OH ou NH-CS-NHR² avec R² représentant un groupe alkyle, un groupe dérivé d'un monosaccharide ou d'un oligo saccharide éventuellement substitué, un groupe dérivé d'un glycosyl-aminoacide ou un groupe dérivé d'un glycopeptide, à condition que l'un au moins des R¹ représente NH-CS-NHR².

2. Thiouréido-cyclodextrine selon la revendication 1, caractérisé en ce que tous les R¹ représentent -NH-CS-NH-R².

3. Thiouréido-cyclodextrine selon la revendication 1, caractérisé en ce que l'un des R¹ représente -NH-CS-NH-R² et les autres R¹ représentent OH.

4. Thiouréido-cyclodextrine selon la revendication 3, caractérisé en ce que R² est le groupe méthyle.

5. Thiouréido-cyclodextrine selon la revendication 2, caractérisé en ce que R² est le groupe méthyl-α-D glucopyranosid-6-yle.

6. Thiouréido-cyclodextrine selon la revendication 3, caractérisé en ce que R² est le groupe méthyl-2,3,4-tri-O-acétyl-α-D-glucopyranosid-6-yle.

7. Thiouréido-cyclodextrine selon la revendication 3, caractérisé en ce que R² est le groupe N-glycyl-β-D glucopyranosylamin-6-yle.

8. Procédé de préparation d'une thiouréido-cyclodextrine répondant à la formule : 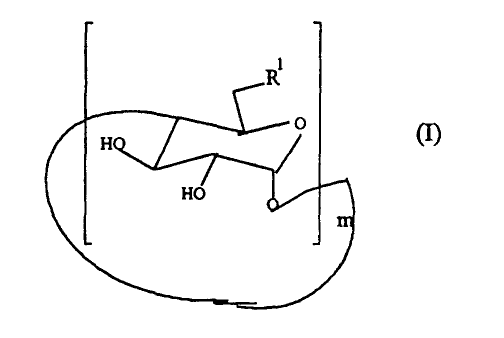 dans laquelle m est égal à 6, 7 ou 8 et les R¹ qui peuvent être identiques ou différents, représentent OH ou NH-CS-NHR² avec R² représentant un groupe alkyle, un groupe dérivé d'un monosaccharide ou d'un oligo saccharide éventuellement substitué, un groupe dérivé d'un glycosyl-aminoacide ou un groupe dérivé d'un glycopeptide, à condition que l'un au moins des R¹ représente NH-CS-NHR²,
caractérisé en ce que l'on fait réagir une isocyanato cyclodextrine de formule : 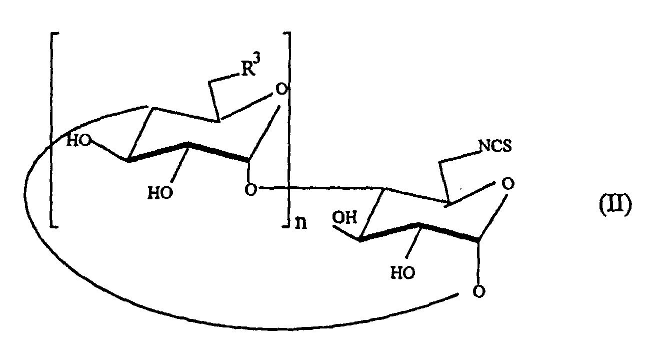 dans laquelle les R³, qui peuvent être identiques ou différents, représentent OH ou NCS, et n est égal à 5, 6 ou 7, avec une amine de formule R²-NH₂ dans laquelle R² à la signification donnée ci-dessus.

9. Procédé de préparation d'une thiouréido-cyclodextrine de formule : 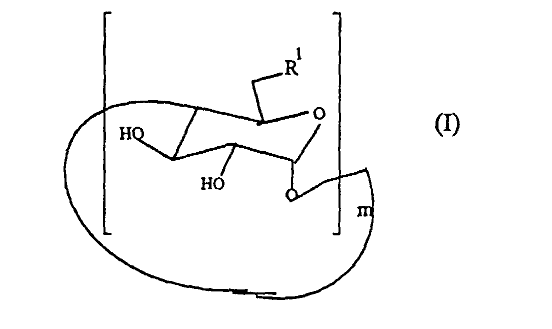 dans laquelle m est égal à 6, 7 ou 8 et les R¹ qui peuvent être identiques ou différents, représentent OH ou NH-CS-NHR² avec R² représentant un groupe alkyle, un groupe dérivé d'un monosaccharide ou d'un oligo saccharide éventuellement substitué, un groupe dérivé d'un glycosyl-aminoacide ou un groupe dérivé d'un glycopeptide, à condition que l'un au moins des R¹ représente NH-CS-NHR²,
caractérisé en ce que l'on fait réagir une amino-cyclodextrine de formule : 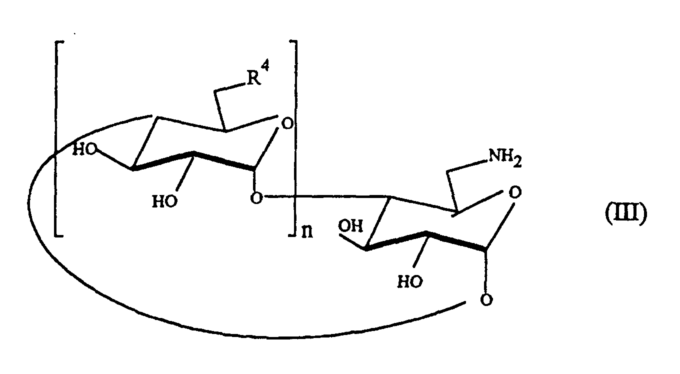 dans laquelle les R⁴, qui peuvent être identiques ou différents, représentent OH ou NH², et n est égal à 5, 6 ou 7, avec un isothiocyanate de formule R²-NCS dans laquelle R² à la signification donnée ci-dessus.

10. Complexe d'inclusion d'une thiouréido-cyclodextrine selon l'une quelconque des revendications 1 à 7 avec une molécule pharmacologiquement active.

11. Complexe selon la revendication 10,
caractérisé en ce que la molécule pharmacologiquement active est un agent antitumoral ou antiparasitaire.

12. Complexe selon la revendication 11,
caractérisé en ce que l'agent antitumoral ou antiparasitaire appartient à la famille du Taxol.

13. Composition pharmaceutique comprenant un complexe d'inclusion d'une thiouréido-cyclodextrine selon l'une quelconque des revendications 10 à 12, avec un véhicule pharmacologiquement acceptable.

14. Composition pharmaceutique selon la revendication 13 sous forme de solution aqueuse.

## Patentansprüche

1. Thioureid-Cyclodextrin mit der Formel: 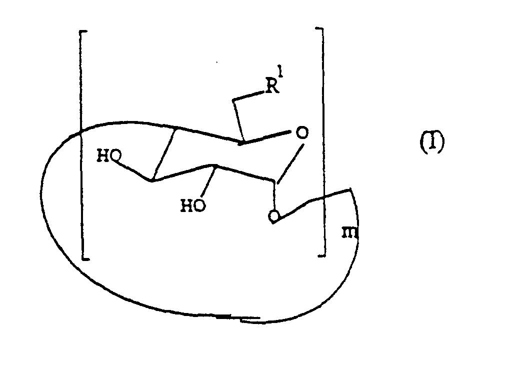 bei der m = 6, 7 oder 8 und bei der die R¹-Komponenten, die identisch oder verschieden sein können, OH oder NH-CS-NHR² aufweisen, wobei R² eine Alkylgruppe, eine aus einem Monosaccharid oder einem eventuell substituierten Oligosaccharid abgeleitete Gruppe, eine aus einer Glycosol-Aminosäure abgeleitete Gruppe oder eine aus einem Glycopeptid abgeleitete Gruppe aufweist, unter der Bedingung, daß mindestens eine der R¹-Komponenten NH-CS-NHR² aufweist.

2. Thioureid-Cyclodextrin gemäß Anspruch 1, dadurch gekennzeichnet, daß alle R¹-Komponenten -NH-CS-NH-R² aufweisen.

3. Thioureid-Cyclodextrin gemäß Anspruch 1, dadurch gekennzeichnet, daß eine der R¹-Komponenten -NH-CS-NH-R² aufweist und die übrigen R¹-Komponenten OH aufweisen.

4. Thioureid-Cyclodextrin gemäß Anspruch 3, dadurch gekennzeichnet, daß R² die Methylgruppe ist.

5. Thioureid-Cyclodextrin gemäß Anspruch 2, dadurch gekennzeichnet, daß R² die Methyl-α-D-Glucopyranosid-6-yl-Gruppe ist.

6. Thioureid-Cyclodextrin gemäß Anspruch 3, dadurch gekennzeichnet, daß R² die Methyl-2,3,4-tri-O-Acetyl-α-D-Glucopyranosid-6-yl-Gruppe ist.

7. Thioureid-Cyclodextrin gemäß Anspruch 3, dadurch gekennzeichnet, daß R² die N-Glyzyl-β-D-Glucopyranosylamin-6-yl-Gruppe ist.

8. Verfahren zur Herstellung eines Thioureid-Cyclodextrins mit der Formel: 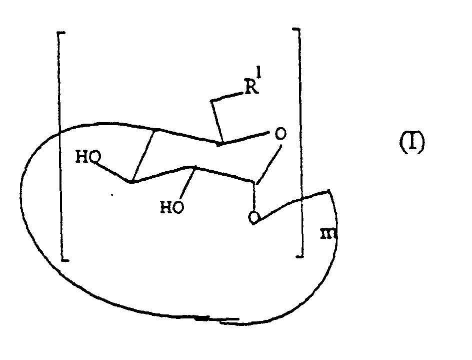 bei der m = 6, 7 oder 8 und bei der die R¹-Komponenten, die identisch oder verschieden sein können, OH oder NH-CS-NHR² aufweisen, wobei R² eine Alkylgruppe, eine aus einem Monosaccharid oder einem eventuell substituierten Oligosaccharid abgeleitete Gruppe, eine aus einer Glycosol-Aminosäure abgeleitete Gruppe oder eine aus einem Glycopeptid abgeleitete Gruppe aufweist, unter der Bedingung, daß mindestens eine der R¹-Komponenten NH-CS-NHR² aufweist,
dadurch gekennzeichnet, daß man ein Isocyanat-Cyclodextrin mit der folgenden Formel zur Reaktion bringt: 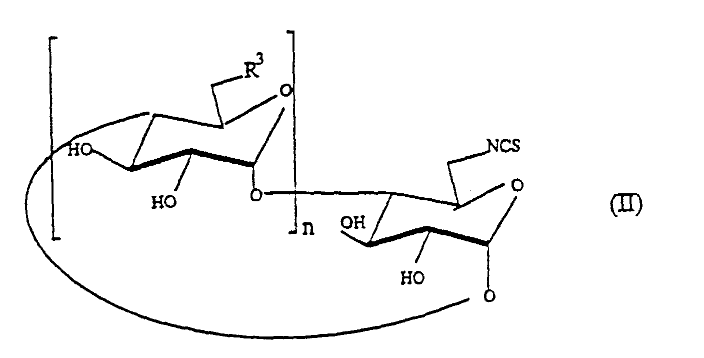 bei der die R³-Komponenten, die identisch oder verschieden sein können, OH oder NCS aufweisen und bei der n = 5, 6 oder 7, mit einem Amin der Formel R²-NH₂, bei der R² die oben angegebene Bedeutung hat.

9. Verfahren zur Herstellung eines Thioureid-Cyclodextrins mit der Formel: 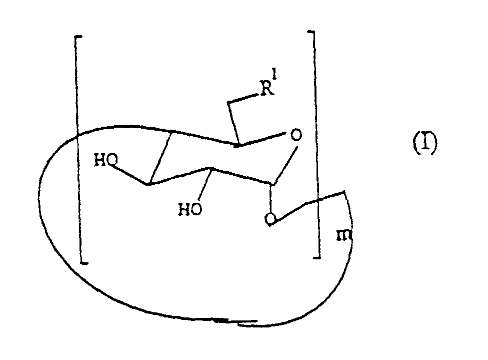 bei der m = 6, 7 oder 8 und bei der die R¹-Komponenten, die identisch oder verschieden sein können, OH oder NH-CS-NHR² aufweisen, wobei R² eine Alkylgruppe, eine aus einem Monosaccharid oder einem eventuell substituierten Oligosaccharid abgeleitete Gruppe, eine aus einer Glycosol-Aminosäure abgeleitete Gruppe oder eine aus einem Glycopeptid abgeleitete Gruppe aufweist, unter der Bedingung, daß mindestens eine der R¹-Komponenten NH-CS-NHR² aufweist,
dadurch gekennzeichnet, daß man ein Amino-Cyclodextrin mit der folgenden Formel zur Reaktion bringt: 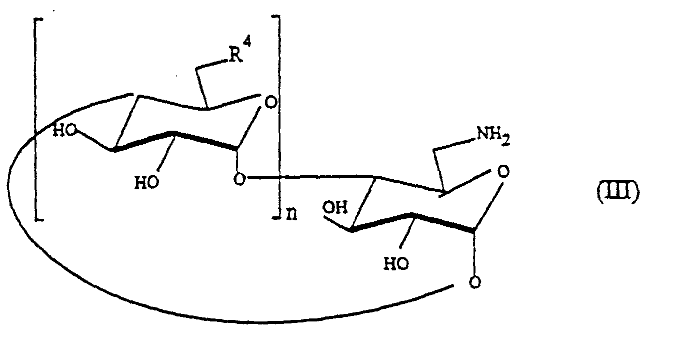 bei der die R⁴-Komponenten, die identisch oder verschieden sein können, OH oder NH₂ aufweisen und bei der n = 5, 6 oder 7, mit einem Isothiocyanat der Formel R²-NCS, bei der R² die oben angegebene Bedeutung hat.

10. Komplex mit Einschluß eines Thioureid-Cyclodextrins gemäß irgendeinem der Ansprüche 1 bis 7 in ein pharmakologisch aktives Molekül.

11. Komplex gemäß Anspruch 10, dadurch gekennzeichnet, daß das pharmakologisch aktive Molekül ein Wirkstoff zur Bekämpfung von Tumoren oder Parasiten ist.

12. Komplex gemäß Anspruch 11, dadurch gekennzeichnet, daß der Wirkstoff zur Bekämpfung von Tumoren oder Parasiten zur Taxol-Gruppe gehört.

13. Pharmazeutische Zusammensetzung, die einen Komplex mit Einschluß eines Thioureid-Cyclodextrins gemäß irgendeinem der Ansprüche 10 bis 12 in einen pharmakologisch akzeptablen Arzneistoffträger enthält.

14. Pharmazeutische Zusammensetzung gemäß Anspruch 13 in Form einer wäßrigen Lösung.

## Claims

1. Thioureido-cyclodextrin corresponding to the formula: 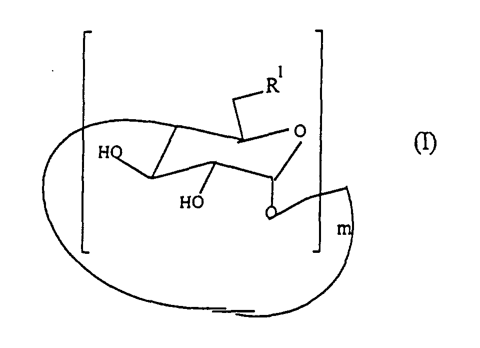 in which m is equal to 6,7 or 8 and where the R¹, which may be identical or different, represent OH or NH-CS-NHR² with R² representing an alkyl group, a group derived from a monosaccharide or oligosaccharide that may be substituted if necessary, a group derived from a glycosyl-amino acid or a group derived from a glycopeptide, provided that at least one of the R¹ represents NH-CS-NHR².

2. Thioureido-cyclodextrin according to claim 1, characterized by the fact that all R¹ represent -NH-CS-NH-R².

3. Thioureido-cyclodextrin according to claim 1, characterized by the fact that one of the R1 represents -NH-CS-NHR² and the others OH.

4. Thioureido-cyclodextrin according to claim 3, characterized by the fact that R2 is the methyl group.

5. Thioureido-cyclodextrin according to claim 2,
characterized by the fact that R² is the methyl-α-D-glucopyranosid-6-yle group.

6. Thioureido-cyclodextrin according to claim 3, characterized by the fact that R² is the methyl-2,3,4-tri-0-acetyl-α-D- glucopyranosid-6-yle group.

7. Thioureido-cyclodextrin according to claim 3,
characterized by the fact that R² is the N-glycyl-β-D glucopyranosylamin-6-yle group.

8. Preparation process of thioureido-cyclodextrin corresponding to the formula: 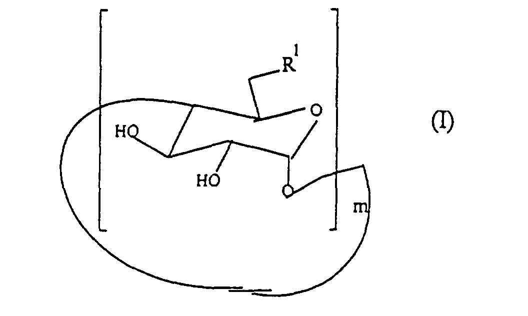 in which m is equal to 6,7 or 8 and where the R¹, which may be identical or different, represent OH or NH-CS NHR² with R² representing an alkyl group, a group derived from a monosaccharide or an oligosaccharide that may be substituted if necessary, a group derived from a glycosyl-amino acid or a group derived from a glycopeptide, provided at least on of the R¹ represents NH-CS-NHR², characterized by the fact that an isocyanato cyclodextrin of formula: 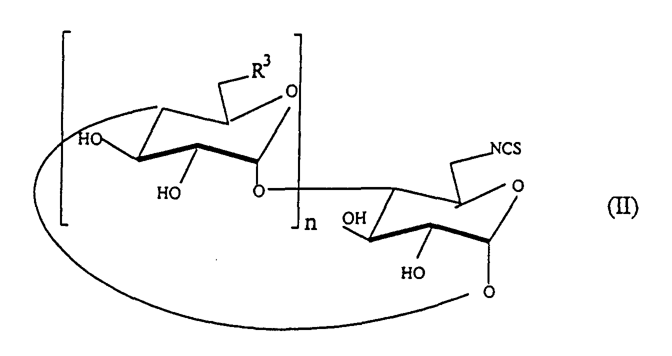 in which the R³, which may be identical or different, represent OH or NCS, and where n is equal to 5, 6 or 7, reacts with an amine of formula R²-NH₂ in which R² corresponds to the signification given below.

9. Preparation process of a thioureido-cyclodextrin of formula: 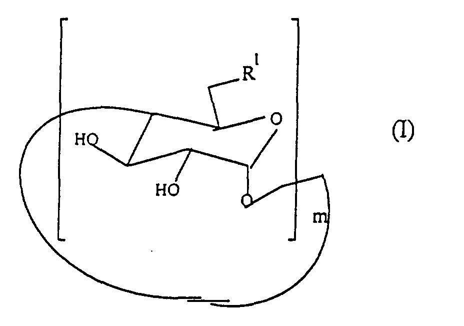 in which m is equal to 6,7, or 8 and where the R¹, which may be identical or different, represent OH or NH-CS-NHR² where R² represents an alkyl group, a group derived from a monosaccharide or oligosaccharide which may be substituted if necessary, a group derived from, a glycosyl-amino acid or a group derived from a glycopeptide, provided at least one of the R¹ represents NH-CS-NHR², characterized by the fact that an aminocyclodextrin of formula: 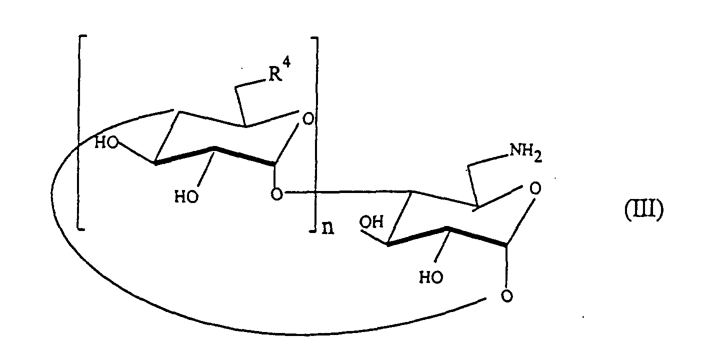 in which the R⁴, which may be identical or different, represent OH or NH2, and where n is equal to 5, 6 or 7, reacts with an isothiocyanate of formula R²-NCS in which R² corresponds to the signification given below.

10. Inclusion complex in a thioureido-cyclodextrin according any one of claims 1 to 7 with a pharmacologically active molecule.

11. Complex according to claim 10, characterized by the fact that the pharmacologically active molecule is an anti-tumor or antiparasitic agent.

12. Complex according to claim 11, characterized by the fact that the anti-tumor or antiparasitic agent belongs to the Taxol family.

13. Pharmaceutical composition comprising an inclusion complex of a thioureido-cyclodextrin according to any one of claims 10 to 12, with a pharmacologically acceptable vector.

14. Pharmaceutical composition according to claim 13 in the form of an aqueous solution.
